# EUROPEAN PATENT SPECIFICATION

(11) **EP 2 834 321 B1**
(45) Date of publication and mention of the grant of the patent: **17.05.2017**
(21) Application number: 13712810.4
(22) Date of filing: 28.03.2013
(51) Int. Cl.: C09K 11/06, H05B 33/14, C07D 405/14, C07D 311/96, H01L 51/00, H01L 51/50

(54) **USE OF A SEMICONDUCTING COMPOUND IN AN ORGANIC LIGHT EMITTING DEVICE**
VERWENDUNG EINER HALBLEITERVERBINDUNG IN EINER ORGANISCHEN LICHTEMITTIERENDEN VORRICHTUNG
UTILISATION D'UN COMPOSÉ SEMI-CONDUCTEUR DANS UN DISPOSITIF ORGANIQUE ÉMETTANT DE LA LUMIÈRE

(30) Priority: 02.04.2012 EP 12162907; 04.01.2013 EP 13150284
(43) Date of publication of application: 11.02.2015
(73) Proprietor: Novaled GmbH, 01307 Dresden (DE)
(72) Inventor: ZÖLLNER, Mike, 01217 Dresden (DE); WUTKE, Jens, 01159 Dresden (DE); FADHEL, Omrane, 01099 Dresden (DE); DENKER, Ulrich, 01307 Dresden (DE)
(74) Representative: Bittner, Thomas L.
(86) International application number: PCT/EP2013/056785
(87) International publication number: WO 2013/149958

(56) References cited:
- CN-A- 101 440 082
- JP-A- 2009 191 232
- LIU F ET AL: "Facile synthesis of spirocyclic aromatic hydrocarbon derivatives based on o-halobiaryl route and Domino reaction for deep-blue organic semiconductors", ORGANIC LETTERS, AMERICAN CHEMICAL SOCIETY, US, vol. 11, no. 17, 3 September 2009 (2009-09-03), pages 3850-3853, XP002610922, ISSN: 1523-7060, DOI: 10.1021/OL900978X [retrieved on 2009-08-05]

## Description

### I. BACKGROUND OF THE INVENTION

Since the demonstration of efficient organic light emitting diodes (OLEDs) by Tang et al. in 1987 (C.W. Tang et al., Appl. Phys. Lett. 51 (12), 913 (1987)), OLEDs developed from a promising candidate technology to high-end commercial displays and luminaires. An OLED comprises a sequence of thin layers substantially made of organic materials between two electrodes. The layers typically have a thickness in the range of 1 nm to 5 µm. The layers are usually formed either in vacuum by means of vapor deposition or from a solution, for example by means of spin-coating or printing.

OLEDs emit light after the injection of charge carriers in a light emitting layer in the form of electrons from the cathode side and in form of holes from the anode side. The charge carrier injection is effected on the basis of an applied external voltage, the subsequent formation of excitons in a light emitting zone and the radiative recombination of those excitons. At least one of the electrodes is transparent or semitransparent, in the majority of cases in the form of a transparent oxide, such as indium tin oxide (ITO), or a thin metal layer.

Flat displays based on OLEDs can be realized both as a passive matrix and as an active matrix. In the case of passive matrix displays, the image is generated by for example, the lines being successively selected and an image information item selected on the columns being represented. However, such displays are restricted to a size of approximately 100 lines for technical construction reasons.

Examples of OLED layer stacks used for displays are described by Duan et al (DOI: 10.1002/adfm.201100943). Duan shows blue OLEDs and white OLEDs. He modified the devices with one light emitting layer to a double and triple light emitting layer, achieving a longer lifetime at the cost of a more complex device stack. Other state-of-the art stacks are disclosed in US6878469 B2, WO 2009/107596 A1 and US 2008/0203905.

Besides display, OLEDs have also been used for lighting, currently available products have efficiency for warm-white of up to 45 lm/W.

The efficiency is being constantly increased, and so is the operational lifetime. With an increase in operational lifetime it is possible to drive the OLEDs with higher current densities and obtain higher luminous intensity per unit area. Even if the devices have high power conversion efficiency, it is still well below 100 %, therefore high current densities imply in a higher operational temperature.

Xie Linghai ( 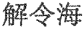 and others describe a spiroxanthene-based material for an OLED with an increased thermal stability (Abstract of CN101440082A). The document describes 6 compounds, including 3 synthesis procedures, however, no concrete data regarding the disclosed compounds (e.g., their glass transition temperatures or melting points) are given. One compound is then used in an emitting layer of an OLED. Lot of various compounds comprising a spiroxanthene core is suggested for an OLED use in the cited document, however, it remains unclear, which of them may be generally useful in electronic devices, specifically, which of them may be suitable in specific functions not directly linked to light emission, e.g. as electron transport layers. Especially uncertain is their general applicability as undoped layers between the emitting layer and the cathode, due to the lack of a deep HOMO for the hole blocking function in the reported compounds. It is also unclear whether at least some of the disclosed compounds allow a successful use in electrically doped layers, especially in combination with technically advantageous molecular dopants having high molecular weight. Moreover, in the light of missing data, it is unclear whether all suggested compounds afford the high thermal stability of the devices using them. Further compounds based on spirofluorene-xanthene, used in an organic light emitting device (OLED) are described in JP2009191232A and by F. Liu et al: "Facile synthesis of spirocyclic aromatic hydrocarbon derivatives based on o-halobiaryl route and Domino reaction for deep-blue organic semiconductors", Organic Letters, American Chemical Society, vol. 11, no. 17, 3 September 2009, pages 3850-3853.

It is an objective of the invention to provide electronic devices with a low operational voltage and better efficiency, specifically, OLEDs with a low operational voltage, good power efficiency and, simultaneously, with good thermal characteristics.

### II. SUMMARY OF THE INVENTION

The problem is solved by use of a compound according to Formula 1: 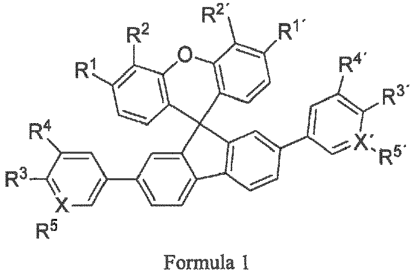
wherein each of R¹, R², R^{1'}, R^{2'} is independently selected from H, C₁₋C₆ alkyl, C₁₋C₆ haloalkyl and C₆-C₁₀ aryl or both substituents on the same aromatic ring of the xanthene skeleton are hydrocarbyl groups linked with each other to form together an anelated divalent C₂-C₁₀ hydrocarbyl group;
X and X' are independently selected from C and N,
R⁵ is H if X is C, R⁵ is H if X*'* is C, R⁵ is lone electron pair if X is N, R^{5*'*} is lone electron pair if X*'* is N, and
each of R³, R⁴, R^{3*'*}, R^{4*'*} is independently selected from H and C₆-C₁₀ aryl, with the proviso that
   - neither both R³, R⁴ nor both R^{3*'*}, R^{4*'*} are aryl at the same time and
   - if X is C, R³ and R⁴ are not H at the same time, and if X' is C, R^{3*'*} and R^{4*'*} are not H at the same time, or
   both substituents on the same phenyl or pyridyl ring are hydrocarbyl groups linked with each other to form together a divalent C₄-C₁₀ hydrocarbyl group representing an anelated, substituted or unsubstituted, six-membered aromatic ring;
   in an electron transporting layer or in an electron injecting layer comprised in an electronic device.

It may be preferred that the electronic device is an organic light emitting device.

It is understood that the alkyl can be straight or branched and may comprise a ring structure. Examples of alkyl substituents are methyl, ethyl, propyl, isopropyl, butyl, cyclopentyl, cyclohexyl. Specific examples of halogenated alkyls are perfluorinated alkyls like trifluoromethyl, perfluorethyl, perfluor-t-butyl. The aryl comprises one aromatic ring and can be substituted or unsubstituted. It is understood that if any substituents are present, they are included in the overall count of the carbon atoms. Examples of aryls are phenyl, tolyl, xylyl, tert-butylphenyl. Preferred are compounds of the formula 1 wherein the substituents having the same denomination differing only by the prime sign, e.g. R¹ and R^{1*'*}, are the same. More preferred is the use of compounds wherein R¹, R², R^{1*'*}, R^{2*'*} is H, or R¹ with R² and R^{1*'*} with R^{2*'*} form anelated benzo-rings. Even preferred is use of compounds wherein, in the formula 1, R³ and R^{3*'*} are selected from H and phenyl, or R³ with R⁴ and R^{3*'*} with R^{4*'*} form anelated benzo-rings. Most preferred is use of compounds having formula 1, wherein R³ and R^{3*'*} are selected from H and phenyl, or R³ with R⁴ and R^{3*'*} with R^{4*'*} form anelated benzo-rings, R¹, R², R^{1*'*}, R^{2*'*} is H and X and X' is C. The term "anelated benzo-ring" can be explained on the compounds C1 and C4 of examples given below. C4 can be seen as a C1 derivative, wherein R¹ with R² and R^{1*'*} with R^{2*'*} form anelated benzo-rings.

A first preferred use of the compound according to Formula 1 is the use in an, preferably organic light emitting, electronic device comprising a first electrode and a second electrode on a substrate, a light emitting layer between the first and the second electrodes, a first electron transporting layer, which is non-light emitting, between the light emitting layer and the first electrode, which first electron transporting layer comprises the compound according to formula 1.

Preferably, the first first electron transporting layer consists of the compound according to formula 1. More preferably, the first electron transporting layer consists of a single species compound. In a preferred mode, the first electron transporting layer is a hole blocking layer, meaning that there is a potential barrier for injection of hole from the light emitting layer to the first electron transport layer. The barrier is sufficiently high to suppress (block) essentially all hole injection from the light emitting layer into the first electron transporting layer under normal operating conditions. It may be provided that the electron transporting layer is thin, with a nominal thickness of less than 50 nm, preferably less than 30 nm.

Alternatively or in addition, the electronic device further comprises a second electron transporting layer between the first electron transporting layer and the first electrode. In a preferred mode, the emitting layer, the first electron transporting layer, and the second electron transporting layer form a consecutive sequence of layers with direct contact between the layers.

In addition, the second electron transporting layer comprises at least 2 different compounds, one of them serving as an electron transporting matrix and another one serving as an electrical dopant. An electrical dopant is a compound improving electrical properties of the matrix in a device, especially its conductivity and/or its charge injection properties.

Preferably, the second electron transporting layer comprises an electron transporting matrix and an electrical dopant. Even preferred, the electron transporting matrix in the second electron transporting layer comprises compound of formula 1. Further preferred, the first electron transporting layer comprises an electrical dopant.

Further object of the invention is an electrically doped semiconducting material comprising at least one electrical dopant and compound of formula 1. The electrical dopant is preferably a redox n-dopant increasing the concentration of electrons which are able to drift in the doped layer in comparison with a layer consisting only of the neat electron transporting matrix.

Further object of the invention is electronic device comprising a compound having formula 1. Another object of the invention is an electronic device comprising the inventive electrically doped semiconducting material. Another object of the invention is electronic device comprising the electrically doped semiconducting material containing a matrix having formula 1. Another object of the invention is a compound having the structure according to generic formula 1.

### III. ADVANTAGEOUS EFFECT OF THE INVENTION

Table 1 summarizes results from device experiments described in more detail in examples. It shows that for the use in electron transporting layers of electronic devices, reasonable selection must be done from the compounds generally proposed for the OLED use in the above mentioned previous art document CN101440082A.

**Table 1**

| code | formula | Voltage (V) (20 nm HBL) | Voltage (V) (10 nm HBL) | T_{g} °C |
|---|---|---|---|---|
| C1 | 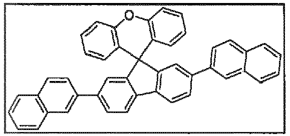 | 4.0 | 3.7 | 121 |
| C2 | 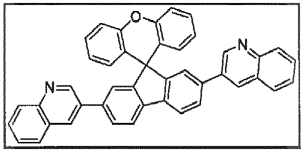 | 4.3 | 4.0 | 136 |
| C3 | 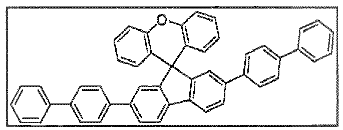 | 4.0 | 3.8 | 142 |
| C4 | 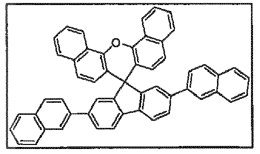 | 4.2 | 3.9 | 164 |
| C5 | 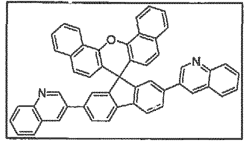 | 4.3 | 4.0 | 176 |
| C6 | 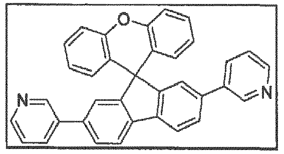 | 4.1 | 3.8 | 113 |
| E1 | 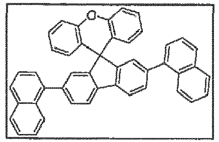 | 5.8 | 5.6 | 118 |
| E3 | 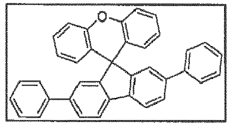 | 5.0 | 4.7 | 105 |

It was surprisingly found that e.g. very similar isomeric compounds C2 and E1 having also very similar LUMO levels (in terms of their redox potentials measured by CV) provide dramatically different performance as electron transporting matrices. Formula 1 thus represents generalization of the structure which provides good electron transporting ability.

On top, Formula 1 compounds offer high glass transition temperatures resulting in high thermal stability of the device.

### IV. BRIEF DESCRIPTION OF THE DRAWINGS

FIG. 1 shows a schematic illustration of a device in which the present invention can be incorporated.
FIG. 2 shows a schematic illustration of a device in which the present invention can be incorporated.
FIG. 3 shows ¹H NMR spectrum of the inventive compound C3
FIG. 4 shows ¹H NMR spectrum of the inventive compound C1
FIG. 5 shows ¹H NMR spectrum of the inventive compound C2
FIG. 6 shows ¹H NMR spectrum of the comparative compound E1
FIG. 7 shows ¹H NMR spectrum of the comparative compound E3
FIG. 8 shows ¹H NMR spectrum of the inventive compound C6
FIG. 9 shows ¹H NMR spectrum of the inventive compound C4
FIG. 10 shows ¹H NMR spectrum of the inventive compound C5
FIG. 11 shows the current density versus applied bias for the inventive and comparative examples.
FIG. 12 shows the luminance intensity density versus applied bias for the inventive and comparative examples.

### V. DETAILED DESCRIPTION OF THE INVENTION

### Device Architecture

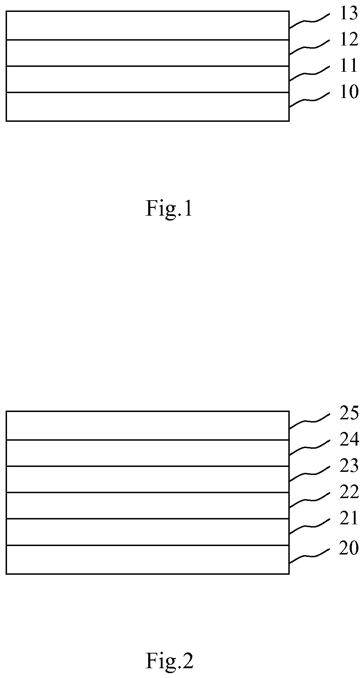

Fig. 1 shows a stack of anode (10), organic semiconducting layer (11) comprising the light emitting layer, electron transporting layer (ETL) (12), and cathode (13). Other layers can be inserted between those depicted, as explained herein.

Fig. 2 shows a stack of an anode (20), a hole injecting and transporting layer (21), a hole transporting layer (22) which can also aggregate the function of electron blocking, a light emitting layer (23), an ETL (24), and a cathode (25). Other layers can be inserted between those depicted, as explained herein.

The wording "device" comprises the organic light emitting diode.

### Material properties - energy levels

A method to determine the ionization potentials (IP) is the ultraviolet photo spectroscopy (UPS). It is usual to measure the ionization potential for solid state materials; however, it is also possible to measure the IP in the gas phase. Both values are differentiated by their solid state effects, which are, for example the polarization energy of the holes that are created during the photo ionization process. A typical value for the polarization energy is approximately 1 eV, but larger discrepancies of the values can also occur. The IP is related to beginning of the photoemission spectra in the region of the large kinetic energy of the photoelectrons, i.e. the energy of the most weakly bounded electrons. A related method to UPS, the inverted photo electron spectroscopy (IPES) can be used to determine the electron affinity (EA). However, this method is less common. Electrochemical measurements in solution are an alternative to the determination of solid state oxidation (Eₒₓ) and reduction (E_{red}) potential. An adequate method is for example the cyclo-voltammetry. A simple rule is used very often for the conversion of red/ox potentials into electron affinities and ionization potential: IP = 4.8 eV + e*Eₒₓ (vs. ferrocenium/ferrocene (Fc⁺/Fc)) and EA = 4.8 eV + e*E_{red} (vs. Fc⁺/Fc) respectively (see B.W. Andrade, Org. Electron. 6, 11 (2005)). Processes are known for the correction of the electrochemical potentials in the case other reference electrodes or other redox pairs are used (see A.J. Bard, L.R. Faulkner, "Electrochemical Methods: Fundamentals and Applications", Wiley, 2. Ausgabe 2000). The information about the influence of the solution used can be found in N.G. Connelly et al., Chem. Rev. 96, 877 (1996). It is usual, even if not exactly correct to use the terms "energy of the HOMO" E(_{HOMO}) and "energy of the LUMO" E(_{LUMO}) respectively as synonyms for the ionization energy and electron affinity (Koopmans Theorem). It has to be taken in consideration, that the ionization potentials and the electron affinities are given in such a way that a larger value represents a stronger binding of a released or respectively of an absorbed electron. The energy scale of the frontier molecular orbitals (HOMO, LUMO) is opposed to this. Therefore, in a rough approximation, is valid: IP = -E(_{HOMO}) and EA = E(_{LUMO}). The given potentials correspond to the solid-state potentials.

### Substrate

It can be flexible or rigid, transparent, opaque, reflective, or translucent. The substrate should be transparent or translucent if the light generated by the OLED is to be transmitted through the substrate (bottom emitting). The substrate may be opaque if the light generated by the OLED is to be emitted in the direction opposite of the substrate, the so called top-emitting type. The OLED can also be transparent. The substrate can be either arranged adjacent to the cathode or anode.

### Electrodes

The electrodes are the anode and the cathode, they must provide a certain amount of conductivity, being preferentially conductors. Preferentially the "first electrode" is the cathode. At least one of the electrodes must be semi-transparent or transparent to enable the light transmission to the outside of the device. Typical electrodes are layers or a stack of layer, comprising metal and/or transparent conductive oxide. Other possible electrodes are made of thin busbars (e.g. a thin metal grid) wherein the spaces between the busbars is filled (coated) with a transparent material with a certain conductivity, such as graphene, carbon nanotubes, doped organic semiconductors, etc.

In one mode, the anode is the electrode closest to the substrate, which is called non-inverted structure. In another mode, the cathode is the electrode closest to the substrate, which is called inverted structure.

Typical materials for the Anode are ITO and Ag. Typical materials for the cathode are Mg:Ag (10 vol.% of Mg), Ag, ITO, Al. Mixtures and multilayer are also possible.

Preferably, the cathode comprises a metal selected from Ag, Al, Mg, Ba, Ca, Yb, In, Zn, Sn, Sm, Bi, Eu, Li, more preferably from Al, Mg, Ca, Ba and even more preferably selected from Al or Mg. Preferred is also a cathode comprising an alloy of Mg and Ag.

### Hole-Transporting Layer (HTL)

Is a layer comprising a large gap semiconductor responsible to transport holes from the anode or holes from a CGL to the light emitting layer (LEL). The HTL is comprised between the anode and the LEL or between the hole generating side of a CGL and the LEL. The HTL can be mixed with another material, for example a p-dopant, in which case it is said the HTL is p-doped. The HTL can be comprised by several layers, which can have different compositions. P-doping the HTL lowers its resistivity and avoids the respective power loss due to the otherwise high resistivity of the undoped semiconductor. The doped HTL can also be used as optical spacer, because it can be made very thick, up to 1000 nm or more without significant increase in resistivity.

### Hole-Injecting Layer (HIL)

Is a layer which facilitates the injection of holes from the anode or from the hole generating side of a CGL into an adjacent HTL. Typically the HIL is a very thin layer (<10 nm). The hole injection layer can be a pure layer of p-dopant and can be about 1 nm thick. When the HTL is doped, an HIL may not be necessary, since the injection function is already provided by the HTL.

### Light-Emitting Layer (LEL)

The light emitting layer must comprise at least one emission material and can optionally comprise additional layers. If the LEL comprises a mixture of two or more materials the charge carrier injection can occur in different materials for instance in a material which is not the emitter, or the charge carrier injection can also occur directly into the emitter. Many different energy transfer processes can occur inside the LEL or adjacent LELs leading to different types of emission. For instance excitons can be formed in a host material and then be transferred as singlet or triplet excitons to an emitter material which can be singlet or triplet emitter which then emits light. A mixture of different types of emitter can be provided for higher efficiency. Mixed light can be realized by using emission from an emitter host and an emitter dopant.

Blocking layers can be used to improve the confinement of charge carriers in the LEL, these blocking layers are further explained in US 7,074,500 B2.

### Electron-Transporting Layer (ETL)

Is a layer comprising a large gap semiconductor responsible to transport electrons from the cathode or electrons from a CGL to the light emitting layer (LEL). The ETL is comprised between the cathode and the LEL or between the electron generating side of a CGL and the LEL. The ETL can be mixed with an electrical n-dopant, in which case it is said the ETL is n-doped. The ETL can be comprised by several layers, which can have different compositions. Electrical n-doping the ETL lowers its resistivity and/or improves its ability to inject electrons into an adjacent layer and avoids the respective power loss due to the otherwise high resistivity (and/or bad injection ability) of the undoped semiconductor. The doped ETL can also be used as optical spacer, because it can be made very thick, up to 1000 nm or more without significant increase in resistivity.

The present invention also employs a compound according to formula 1 in the ETL, which compound can be used in combination with other materials, in the whole layer or in a sublayer of the ETL.

Hole blocking layers and electron blocking layers can be employed as usual.

In one mode of the invention the ETL comprises 2 layers, the first ETL (ETL1) and the second ETL (ETL2), ETL1 is closer to the LEL than the ETL2. Preferentially ETL1 comprises the compound according to formula 1, even more preferably consists only of material according to formula 1. Also preferably, ETL1 is closer to the substrate than ETL2.

Alternatively or in addition, the ETL2 comprises a compound according to formula 1. Preferably, the ETL2 is electrically doped.

Optionally ETL1 and ETL2 comprise the same compound according to formula 1.

Other layers with different functions can be included, and the device architecture can be adapted as known by the skilled in the art. For example, an Electron-Injecting Layer (EIL) can be used between the cathode and the ETL. Also the EIL can comprise the inventive matrix compounds of the present application.

### Charge generation layer (CGL)

The OLED can comprise a CGL which can be used in conjunction with an electrode as inversion contact, or as connecting unit in stacked OLEDs. A CGL can have the most different configurations and names, examples are pn-junction, connecting unit, tunnel junction, etc. Best examples are pn junctions as disclosed in US 2009/0045728 A1 US 2010/0288362 A1. Metal layers and or insulating layers can also be used.

### Stacked OLEDs

When the OLED comprises two or more LELs separated by CGLs, the OLED is named a stacked OLED, otherwise it is named a single unit OLED. The group of layers between two closest CGLs or between one of the electrodes and the closest CGL is named a electroluminescent unit (ELU). Therefore a stacked OLED can be described as anode/ELU₁/{CGL_{X}/ELU_{1+X}}_{X}/cathode, wherein x is a positive integer and each CGL_{X} or each ELU_{1+X} can be equal or different. The CGL can also be formed by the adjacent layers of two ELUs as disclosed in US2009/0009072 A1 Further stacked OLEDs are explained e.g. in US 2009/0045728 A1, US 2010/0288362 A1, and references therein.

### Deposition of Organic Layers

Any organic semiconducting layers of the inventive display can be deposited by known techniques, such as vacuum thermal evaporation (VTE), organic vapour phase deposition, laser induced thermal transfer, spin coating, blade coating, slot dye coating, inkjet printing, etc. A preferred method for preparing the OLED according to the invention is vacuum thermal evaporation.

Preferably, the ETL is formed by evaporation. When using an additional material in the ETL, it is preferred that the ETL is formed by co-evaporation of the electron transporting matrix (ETM) and the additional material. The additional material may be mixed homogeneously in the ETL. In one mode of the invention, the additional material has a concentration variation in the ETL, wherein the concentration changes in the direction of the thickness of the stack of layers. It is also foreseen that the ETL is structured in sub-layers, wherein some but not all of these sub-layers comprise the additional material.

### Electrical doping

The present invention can be used in addition or in combination with electrical doping of organic semiconducting layers.

The most reliable and at the same time efficient OLEDs are OLEDs comprising electrically doped layers. Generally, the electrical doping means improving of electrical properties, especially the conductivity and/or injection ability of a doped layer in comparison with neat charge-transporting matrix without a dopant. In the narrower sense, which is usually called redox doping or charge transfer doping, hole transport layers are doped with a suitable acceptor material (p-doping) or electron transport layers with a donor material (n-doping), respectively. Through redox doping, the density of charge carriers in organic solids (and therefore the conductivity) can be increased substantially. In other words, the redox doping increases the density of charge carriers of a semiconducting matrix in comparison with the charge carrier density of the undoped matrix. The use of doped charge-carrier transport layers (p-doping of the hole transport layer by admixture of acceptor-like molecules, n-doping of the electron transport layer by admixture of donor-like molecules) in organic light-emitting diodes is, e.g., described in US 2008/203406 and US 5,093,698.

US2008227979 discloses in detail the charge-transfer doping of organic transport materials, with inorganic and with organic dopants. Basically, an effective electron transfer occurs from the dopant to the matrix increasing the Fermi level of the matrix. For an efficient transfer in a p-doping case, the LUMO energy level of the dopant is preferably more negative than the HOMO energy level of the matrix or at least slightly more positive, not more than 0.5 eV, to the HOMO energy level of the matrix. For the n-doping case, the HOMO energy level of the dopant is preferably more positive than the LUMO energy level of the matrix or at least slightly more negative, not lower than 0.5 eV, to the LUMO energy level of the matrix. It is further more desired that the energy level difference for energy transfer from dopant to matrix is smaller than + 0.3 eV.

Typical examples of known redox doped hole transport materials are: copperphthalocyanine (CuPc), which HOMO level is approximately -5.2 eV, doped with tetrafluorotetracyanoquinonedimethane (F4TCNQ), which LUMO level is about -5.2 eV; zincphthalocyanine (ZnPc) (HOMO = -5.2 eV) doped with F4TCNQ; a-NPD (N,N'-Bis(naphthalen-1-yl)-N,N'-bis(phenyl)-benzidine) doped with F4TCNQ. a-NPD doped with 2,2'-(perfluoronaphthalene-2,6-diylidene) dimalononitrile (PD1). a-NPD doped with 2,2',2"-(cyclopropane-1,2,3-triylidene)tris(2-(p-cyanotetrafluorophenyl)acetonitrile) (PD2). All p-doping in the device examples of the present application was done with 5 mol.% of PD2.

Typical examples of known redox doped electron transport materials are: fullerene C60 doped with acridine orange base (AOB); perylene-3,4,9,10-tetracarboxylic-3,4,9,10-dianhydride (PTCDA) doped with leuco crystal violet; 2,9 - di (phenanthren-9-yl) - 4,7 - diphenyl - 1,10 - phenanthroline doped with tetrakis (1,3,4,6,7,8 - hexahydro - 2H - pyrimido [ 1,2 - a] pyrimidinato) ditung-sten (II) (W₂(hpp)₄); naphthalene tetracarboxylic acid di-anhydride (NTCDA) doped with 3,6-bis-(dimethyl amino)-acridine; NTCDA doped with bis(ethylenedithio) tetrathiafulvalene (BEDT-TTF).

In the present invention, electrical doping of the inventive ETL matrices with strongly reducing n-dopants is preferred. More preferred are molecular n-dopants having relative molecular weight above 200, wherein a diffusion of the dopant into adjacent layers is substantially suppressed. Most preferred n-dopants in the electrically doped semiconducting material according to the invention are strongly reducing metal complexes like W₂(hpp)₄, having their redox potential, measured by cyclic voltammetry (CV) in THF vs. Fc+/Fc standard, below (in other words, more negative than) - 2.0 V.

Preferred ETL matrix compounds of the present invention are 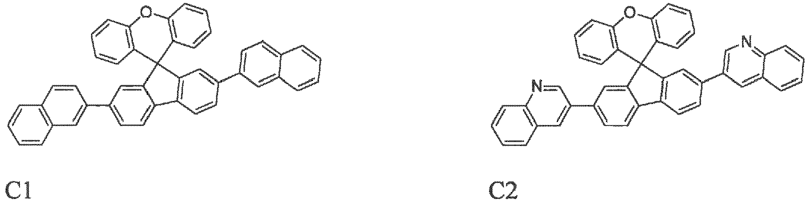 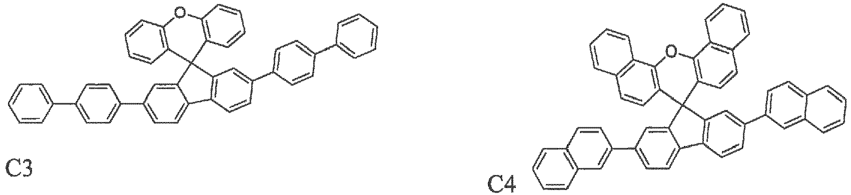 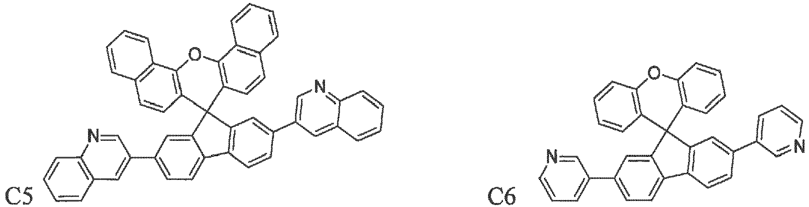 C3 and C1 are the most preferred compounds.

### VI. EXAMPLES

### Synthesis procedures

All manipulations were carried out under argon in thoroughly dried glass vessels, without any further purification of commercial chemicals except for the use of dried and degassed solvents (solvent purification system (SPS) quality). ¹H-NMR spectra were taken at 500.13 MHz, and referenced to 5.31 ppm.

### 1.1 Synthesis procedures for spiroxanthenes

### General synthesis route

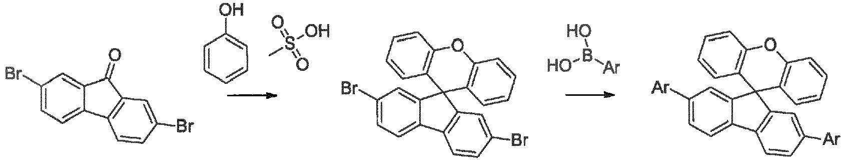

### Synthesis of 2,7-dibromospiro[fluorene-9,9'-xanthene]

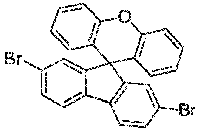

In a flask 2,7-dibromo-9*H-*fluoren-9-one (50.0 g, 147.9 mmol, 1.0 eq) and phenol (134.2 g, 1.43 mol, 9.6 eq) were combined. Methanesulfonic acid (56.9 g, 592 mmol, 4.0 eq) was added. The mixture was stirred for four days at 135 °C and cooled afterwards. 500 mL water and 300 mL dichloromethane were added. The mixture was stirred at room temperature for an hour. A light solid precipitated, which was filtered and washed with methanol until the filtrate was colourless. The solid was triturated in 200 mL hot ethanol and filtered while it was still hot. The solid was washed with ethanol and dried under reduced pressure afterwards.

The target compound was yielded as a white solid 44.4 g (61 %).
Melting point: 265 °C (TGA-DSC, peak)

### Synthesis of 2',7'-dibromospiro[dibenzo[c,h]xanthene-7,9'-fluorene]

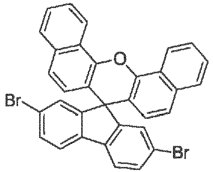

In an inert argon atmosphere methanesulfonic acid (7.7 mL, 11.40 g, 4.0 eq, 118.6 mmol) was added in one portion to a mixture of naphthalen-1-ol (17.06 g, 4.0 eq, 118.3 mmol) and 2,7-dibromo-9*H-*fluoren-9-one (10.00 g, 1.0 eq, 29.6 mmol). The mixture was heated to 150°C over 21 hours maintaining the argon atmosphere. After cooling down to room temperature water (200 mL) and dichloromethane (500 mL) were added, the layers were separated and the organic layer was washed with water (1x 100 mL) and dried over magnesium sulphate.

The solvent was removed *in vacuo* and the remaining residue was taken in ethyl acetate (400 mL), refluxed over 5 minutes and the solid was separated by filtration. After washing with hot ethyl acetate (1x 100 mL) and drying 2',7'-dibromospiro[dibenzo[*c,h*]xanthene-7,9'-fluorene] was obtained.
Yield: 13.9g(79%)
Melting point: 403 °C (TGA-DSC, peak)

Other compounds according to formula 1 with R¹, R², R^{1*'*}, R^{2*'*} being another moiety than hydrogen or anelated benzo-ring can be easily prepared by utilizing respectively substituted phenols, for example by using 3-alkylphenol to prepare 2,7-dibromo-3',6'-di-alkylspiro[fluorene-9,9'-xanthene].

### General procedure for Suzuki-couplings

Either 2,7-Dibromospiro[fluorene-9,9'-xanthene] or 2',7'-dibromospiro[dibenzo[c,h]xanthene-7,9'-fluorene] and boronic acid corresponding to the desired product were combined in a flask, which was evacuated afterwards. After refilling with argon tetrakis(triphenylphosphin)-palladium(0) and toluene were added. Finally the degassed aqueous 2M potassium carbonate solution was added dropwise. The mixture was heated to 85 °C for 19 hours. Reaction was complete as proven by thin layer chromatography (TLC).

The mixture was cooled to room temperature. Unless otherwise stated, water (2 parts) and ethylacetate (3 parts) were added. The mixture was stirred vigorously for 30 min. The solid was filtered and washed with methanol until the filtrate was colourless. The solid was triturated with ethylacetate. After drying in vacuum a grayish solid was obtained. Further purification was accomplished by gradient high vacuum sublimation successfully. The compounds were obtained as white to pale yellow, crystalline solids.

### 2,7-di([1,1'-biphenyl]-4-yl)spiro[fluorene-9,9'-xanthene](C3)

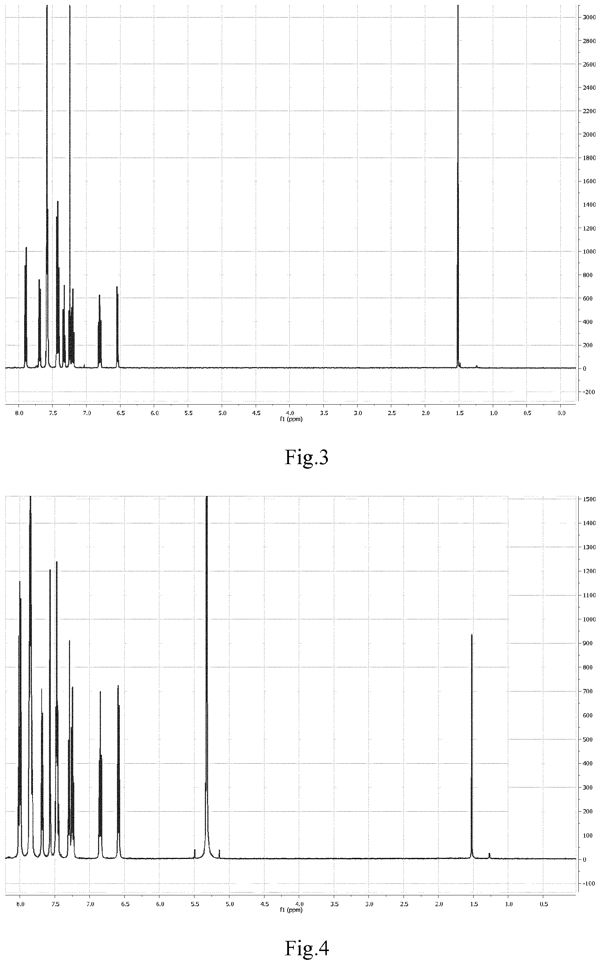

2,7-Dibromospiro[fluorene-9,9'-xanthene]: 20.0 g (1.0 eq, 40.8 mmol)
1,1'-biphenyl-4-yl- boronic acid: 16.2 g (2.0 eq, 81.6 mmol)
Pd(PPh₃)₄: 2.83 g (6 mol.%, 2.45 mmol)
2M K₂CO₃: 45.1 g (8.0 eq, 326.4 mmol)
toluene: 400 mL
Yield: 25.8 g (99 %) grayish solid prior sublimation; after sublimation: white, crystaline solid
Melting point: 297 °C (differential scanning calorimetry (DSC), peak)
Glas transition: 142 °C (DSC, onset)
Cyclic voltammetry: reduction at -2.63 V vs. Fc⁺/Fc in THF
¹H-NMR (CDCl₃) is shown in Fig 3.

### 2,7-di(naphthalen-2-yl)spiro[fluorene-9,9'-xanthene](C1)

2,7-Dibromospiro[fluorene-9,9'-xanthene]: 21.66 g (1.0 eq, 44.19 mmol)
naphthalen-2-yl- boronic acid: 15.2 g (2.0 eq, 88.38 mmol)
Pd(PPh₃)₄: 3.06 g (6 mol.%, 2.65 mmol)
2M K₂CO₃: 176 mL
toluene: 400 mL
Yield: 21.2 g (82 %) grayish solid prior sublimation; after sublimation: white, crystaline solid
Melting point: 297 °C (DSC, peak)
Glas transition: 121 °C (DSC, onset)
Cyclic voltammetry: reduction at -2.63 V vs. Fc⁺/Fc in THF
   ¹H-NMR (CD₂Cl₂) is shown on Fig.4.

### 2,7-di(quinolin-3-yl)spiro[fluorene-9,9'-xanthene] (C2)

2,7-Dibromospiro[fluorene-9,9'-xanthene]: 10,0 g (1.0 eq, 20.4 mmol)
quinoline-3-yl- boronic acid: 8,82 g (2.5 eq, 51.0 mmol)
Pd(PPh₃)₄: 1.41 g (10 mol.%, 1.22 mmol)
2M K₂CO₃: 83 mL
toluene: 200 mL

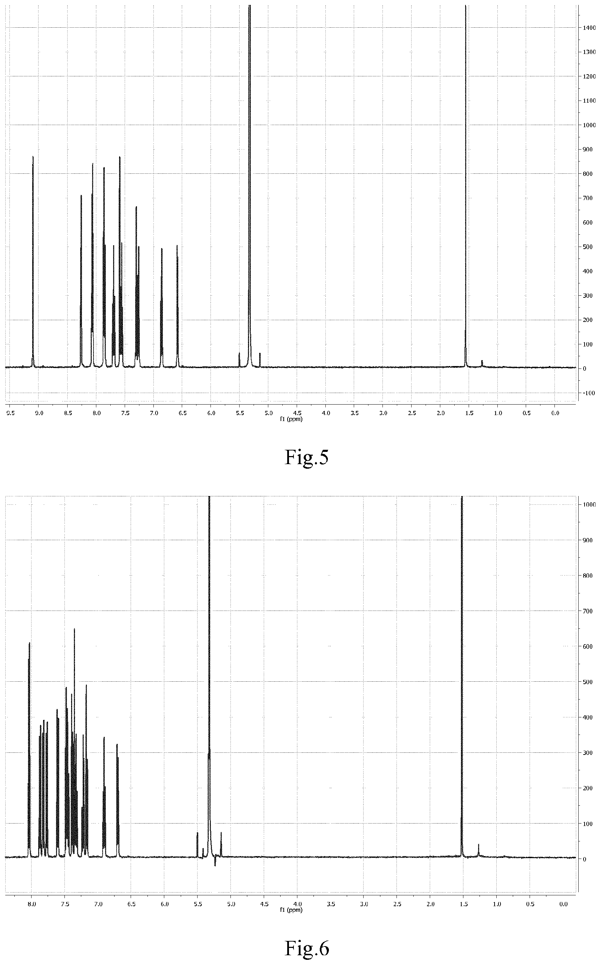

The grayish solid obtained after work up was additionally triturated in hot ethylacetate (100 mL), methanol (100 mL) and dichloromethane (200 mL) subsequently. After filtration the solid was solved in toluene and filtered over Celite. Evaporation of the solvent lead to a precipitate. It was filtered and dried in vacuum. The product was obtained as yellowish solid.
Yield: 5.45 g (46 %) pale yellow solid prior sublimation; after sublimation: pale yellow solid
Melting point: 367 °C (DSC, peak)
Glas transition: 136 °C (DSC, onset)
Cyclic voltammetry: reduction at -2.45 V vs. Fc⁺/Fc in THF
¹H-NMR (CD₂Cl₂): see Fig. 5

### 2,7-di(naphthalen-1-yl)spiro[fluorene-9,9'-xanthene] (E1)

2,7-Dibromospiro[fluorene-9,9'-xanthene]: 3.0 g (1.0 eq, 6.1 mmol)
naphthalen-1-yl- boronic acid: 2.63 g (2.5 eq, 15.3 mmol)
Pd(PPh₃)₄: 424 mg (6 mol.%, 0.37 mmol)
2M K₂CO₃: 25 mL
toluene: 60 mL

The grayish solid obtained after work up was additionally filtered through a pad of Celite with hot chloroform. After evaporation of the solvents, the solid was triturated with hot ethylacetate, filtered and dried under reduced pressure. The product was obtained as white solid.
Yield: 2.92 g (82 %) white solid prior sublimation; after sublimation: white, crystaline solid
Melting point: 331 °C (DSC, peak)
Glas transition: 118 °C (DSC, onset)
Cyclic voltammetry: reduction at -2.78 V vs. Fc⁺/Fc in THF
¹H-NMR (CD₂Cl₂): see Fig. 6

### 2,7-diphenylspiro[fluorene-9,9'-xanthene](E3)

2,7-Dibromospiro[fluorene-9,9'-xanthene]: 20.0 g (1.0 eq, 40.8 mmol)
phenylboronic acid: 9.95 g (2.0 eq, 81.6 mmol)
Pd(PPh₃)₄: 2.83 g (6 mol.%, 2.45 mmol)
2M K₂CO₃: 163 mL
toluene: 400 mL

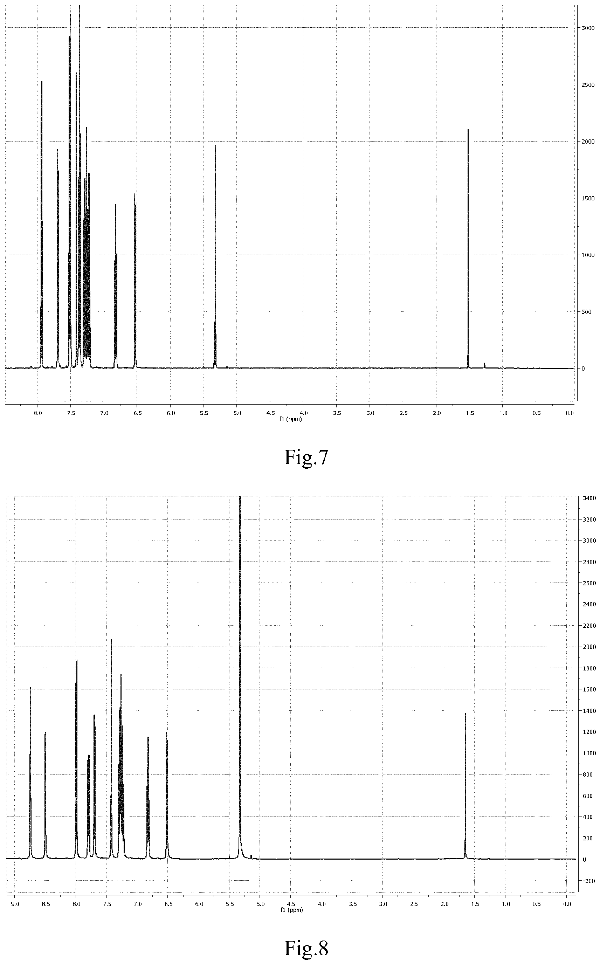

After addition of water and ethylacetate no precipitate was observed. The organic layer was separated and the aqueous one extracted with ethylacetate two times. The combined organic phases were evaporated and the retained solid was triturated with methanol and ethylacetate subsequently.
Yield: 15.3 g (77 %) grayish solid prior sublimation; after sublimation: white, crystaline solid
Melting point: 216 °C (DSC, peak)
Glas transition: 105 °C (DSC, onset)
Cyclic voltammetry: reduction at -2.75 V vs. Fc. in THF
¹H-NMR (CD₂Cl₂): see Fig. 7

### 2,7-dipyridin-3-ylspiro[fluorene-9,9'-xanthene] (C6)

2,7-Dibromospiro[fluorene-9,9'-xanthene]: 4.94 g (1.0 eq, 10 mmol)
3-(4,4,5,5-Tetramethyl-1,3,2-dioxaborolan-2-yl)pyridine: 4.51 g (2.2 eq, 22 mmol)
Pd(PPh₃)₄: 0.69 g (6 mol.%, 0.6 mmol)
2M K₂CO₃: 40 mL
toluene: 100 mL
Yield: 2.85 g (59 %) white solid prior sublimation; after sublimation: white solid
Melting point: 284 °C (DSC, peak)
Glas transition: 113 °C (DSC, onset)
Cyclic voltammetry: reduction at -2.60 V vs. Fc⁺/Fc in THF
¹H-NMR (CD₂Cl₂): see Fig. 8

### 2',7'-di(naphthalen-2-yl)spiro[dibenzo[c,h]xanthene-7,9'-fluorene] (C4)

2',7'-dibromospiro[dibenzo[c,h]xanthene-7,9'-fluorene]: 6.72 g (1.0 eq, 10 mmol)
naphthalen-2-yl- boronic acid: 3.92 g (2.0 eq, 22.8 mmol)
Pd(PPh₃)₄: 0.79 g (6 mol.%, 0.68 mmol)
2M K₂CO₃: 46 mL
toluene: 115 mL

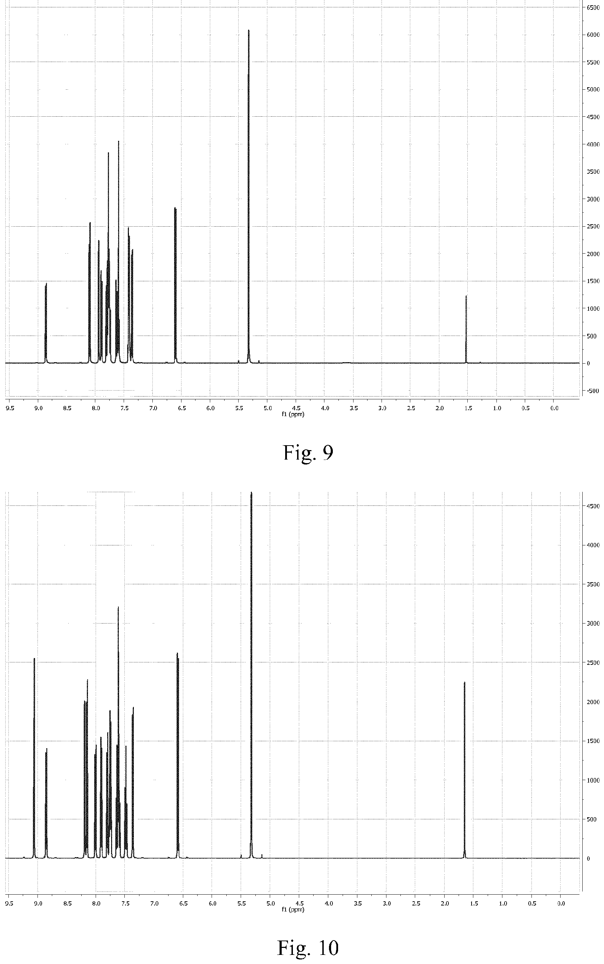

After addition of water the mixture was extracted with dichloromethane three times. The combined organic phases were dried over MgSO₄ and reduced to dryness. After purification via column chromatography (SiO₂, hexane:dichloromethane 2:1) the product was obtained.
Yield: 5.25 g (67 %) pale yellow solid prior sublimation; after sublimation: pale yellow solid
Melting point: 293 °C (TGA-DSC, peak)
Glas transition: 164 °C (DSC, onset)
Cyclic voltammetry: reduction at -2.64 V vs. Fc⁺/Fc in THF
¹H-NMR (CD₂Cl₂): see Fig. 9

### 2',7'-di(quinolin-3-yl)spiro[dibenzo[c,h]xanthene-7,9'-fluorene] (C5)

2',7'-dibromospiro[dibenzo[c,h]xanthene-7,9'-fluorene]: 2.0 g (1.0 eq, 3.4 mmol)
quinoline-3-yl- boronic acid: 1,47 g (2.5 eq, 8.5 mmol)
Pd(PPh₃)₄: 0.23 g (6 mol.%, 0.2 mmol)
2M K₂CO₃: 14 mL
toluene: 35 mL

After addition of water the mixture was extracted with dichloromethane three times. The combined organic phases were dried over MgSO₄ and reduced to dryness. After purification via column chromatography (SiO₂ ethylacetate) the product was obtained.
Yield: 1.62 g (70 %) grayish solid prior sublimation; after sublimation: pale yellow solid
Melting point: 282 °C (DSC, peak)
Glas transition: 176 °C (DSC, onset)
Cyclic voltammetry: reduction at -2.42 V vs. Fc⁺/Fc in THF
¹H-NMR (CD₂Cl₂): see Fig. 10

### Example 1

A bottom emitting blue OLED was fabricated on a glass substrate coated with patterned ITO (90 nm thickness), with the following layer sequence:
1. p-doped N,N'-Bis(naphthalen-1-yl)-N,N'-bis(phenyl)-benzidine (a-NPD) (5 mol.% of 2,2',2"-(cyclopropane-1,2,3-triylidene)tris(2-(p-cyanotetrafluorophenyl)acetonitrile) (PD2) as hole injection and transporting layer with thickness of 130 nm;
2. undoped 4,4',4"-tris(carbazol-9-yl)-triphenylamine (TCTA) with thickness of 10 nm;
3. emitter layer with TPBI:Firpic (molar ratio 4:1) with thickness of 15 nm. TPBI is 1,3,5-tris(1-phenyl-1H-benzimidazol-2-yl)benzene and Firpic is bis(3,5-difluoro-2-(2-pyridyl-(2-carboxypyridyl)iridium(III);
4. 20 nm TPBI;
5. 40 nm of compound C1 doped with tetrakis(1,3,4,6,7,8-hexahydro-2H-pyrimido[1,2-a]pyrimidinato)ditungsten (II) (W₂(hpp)₄) (70:30 mol.%);
6. a cathode of 100 nm Al.

The device was encapsulated with a glass cover containing getter. The voltage at 10 mA/cm² is 4.0 V and the device achieves 1000 cd/m² at a voltage of 4.3 V.

### Example 2

A second OLED was fabricated as in example 1, except that the compound C1 was replaced by compound C2. The voltage at 10 mA/cm² is 4.3 V and the device achieves 1000 cd/m² at a voltage of 4.8 V.

### Example 3

A third OLED was fabricated as in example 1, except that the compound C1 was replaced by compound C3. The voltage at 10 mA/cm² is 4.0 V and the device achieves 1000 cd/m² at a voltage of 4.2 V.

### Comparative Example 1

A comparative OLED was fabricated as in example 1, except that the compound C1 was replaced by 2,7-di(naphthalen-1-yl)spiro[fluorene-9,9'-xanthene] (E1). The voltage at 10 mA/cm² is 5.8 V and the device achieves 1000 cd/m² at a voltage of 6.5 V, which is considerably higher than for the examples according to the invention.

An additional comparative OLED was fabricated as in example 1, except that the compound C1 was replaced by 2,7-diphenylspiro[fluorene-9,9'-xanthene] (E3). The voltage at 10 mA/cm² is 5.0 V and the device achieves 1000 cd/m² at a voltage of 5.6 V, which is considerably higher than for the examples according to the invention.

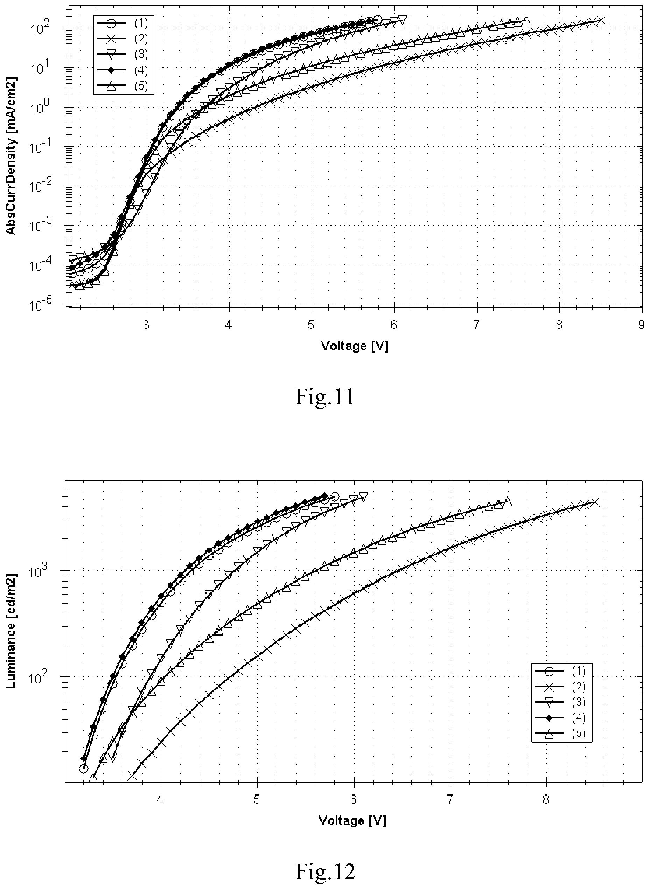

Fig. 11 shows the current density versus the applied bias of above described devices. (1) refers to compound C1, (2) refers to compound E1, (3) refers to compound C2, (4) refers to compound C3 and (5) refers to compound E3. It can be seen that there is a dramatic difference in the current magnitude between the inventive compounds C1, C2, C3 on one side and comparative compounds E1 and E3 on the other side, of more than one order of magnitude, in the range of typical operating voltages.

Fig. 12 shows the density of the luminous intensity versus the applied bias of above described devices. (1) refers to compound C1, (2) refers to compound E1, (3) refers to compound C2, (4) refers to compound C3 and (5) refers to compound E3. It can be seen that there is a dramatic difference of the inventive compounds in relation to the comparative examples. The working and comparative examples were repeated with the same arrangement with the TPBI blocking layer having thickness 10 nm. The results are summarized in Table 1. The inventive compounds clearly show an unexpected advantage over comparative compounds which either have structural similarity or equal LUMOs or even both. The similar LUMO levels should make the inventive and comparative compounds similarly dopable. Nevertheless, the inventive compounds show unexpectedly good dopability in comparison with the structurally as well as functionally (in terms of the LUMO level) closest comparative compounds. The practical applicability of this surprising finding was successfully demonstrated through improved performance of OLED devices comprising inventive compounds.

### Used abbreviations

- CV: cyclovoltammetry
- DCM: dichloromethan
- DSC: differential scanning calorimetry
- Fc⁺/Fc: ferrocenium/ferrocene reference system
- HPLC: high performance liquid chromatography
- SPS: solvent purification system
- TGA: thermogravimetry thermal analysis
- THF: tetrahydrofuran
- TLC: thin layer chromatography
- UV: UV/Vis spectroscopy
- eq: chemical equivalent
- mol.%: molar percent
- vol.%: volume percent

## Claims

1. Use of a compound according to formula 1: 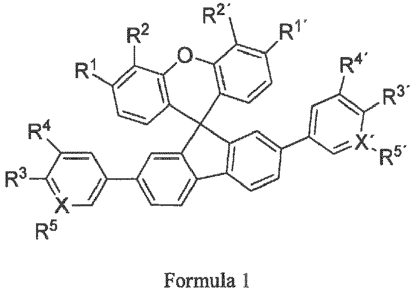
wherein each of R¹, R², R¹, R^{2*'*} is independently selected from H, C₁-C₆ alkyl, C₁-C₆ haloalkyl and C₆-C₁₀ aryl or both substituents on the same aromatic ring of the xanthene skeleton are hydrocarbyl groups linked with each other to form together an anelated divalent C₂-C₁₀ hydrocarbyl group;
X and X' are independently selected from C and N,
R⁵ is H if X is C, R⁵ is H if X*'* is C, R⁵ is lone electron pair if X is N, R^{5*'*} is lone electron pair if X*'* is N, and
each of R³, R⁴, R^{3*'*}, R^{4*'*} is independently selected from H and C₆-C₁₀ aryl, with the proviso that
- neither both R³, R⁴ nor both R^{3*'*}, R^{4*'*} are aryl at the same time and
- if X is C, R³ and R⁴ are not H at the same time, and if X' is C, R^{3*'*} and R^{4*'*} are not H at the same time, or
both substituents on the same phenyl or pyridyl ring are hydrocarbyl groups linked with each other to form together a divalent C₄-C₁₀ hydrocarbyl group representing an anelated, substituted or unsubstituted, six-membered aromatic ring;
in an electron transporting layer or in an electron injecting layer comprised in an electronic device.

2. Use according to claim 1, wherein the electronic device comprises a first electrode and a second electrode on a substrate, a light emitting layer between the first and the second electrodes, a first electron transporting layer between the light emitting layer and the first electrode, which first electron transporting layer comprises the compound according to formula 1.

3. Use according to claim 2, wherein the first electron transporting layer consists of the compound according to formula 1.

4. Use according to claims 3, wherein the first electron transporting layer consists of a single species compound.

5. Use according to any of the claims 2 to 4, wherein the first electron transporting layer is a hole blocking layer.

6. Use according to any of the previous claims 2 to 5, wherein the electronic device further comprises a second electron transporting layer between the first electron transporting layer and the first electrode.

7. Use according to claim 6, wherein the second electron transporting layer comprises an electron transporting matrix and an electrical dopant.

8. Use according to claim 7, wherein the electron transporting matrix in the second electron transporting layer comprises compound of formula 1.

9. Use according to any of the claims 2 to 8, wherein the first electron transporting layer comprises an electrical dopant.

10. Electrically doped semiconducting material comprising at least one electrical dopant and compound of formula 1 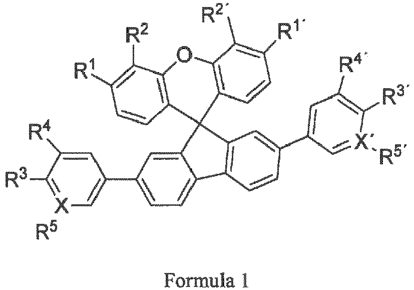
wherein each of R¹, R², R¹, R^{2*'*} is independently selected from H, C₁-C₆ alkyl, C₁-C₆ haloalkyl and C₆-C₁₀ aryl or both substituents on the same aromatic ring of the xanthene skeleton are hydrocarbyl groups linked with each other to form together an anelated divalent C₂-C₁₀ hydrocarbyl group;
X and X' are independently selected from C and N,
R⁵ is H if X is C, R^{5*'*} is H if X*'* is C, R⁵ is lone electron pair if X is N, R^{5*'*} is lone electron pair if X*'* is N, and
each of R³, R⁴, R³, R^{4*'*} is independently selected from H and C₆-C₁₀ aryl, with the proviso that
- neither both R³, R⁴ nor both R^{3*'*}, R^{4*'*} are aryl at the same time and
- if X is C, R³ and R⁴ are not H at the same time, and if X*'* is C, R^{3*'*} and R^{4*'*} are not H at the same time, or
both substituents on the same phenyl or pyridyl ring are hydrocarbyl groups linked with each other to form together a divalent C₄-C₁₀ hydrocarbyl group representing an anelated, substituted or unsubstituted, six-membered aromatic ring.

11. Electronic device comprising the electrically doped semiconducting material of claim 10.

12. Compound having the structure according generic formula 1 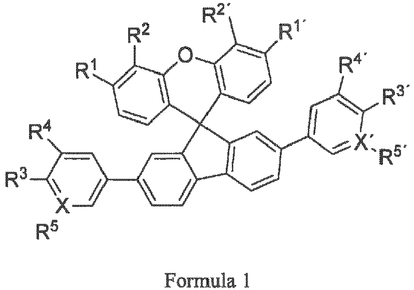
wherein each of R¹, R², R^{1*'*}, R^{2*'*} is independently selected from H, C₁-C₆ alkyl, C₁-C₆ haloalkyl and C₆-C₁₀ aryl or both substituents on the same aromatic ring of the xanthene skeleton are hydrocarbyl groups linked with each other to form together an anelated divalent C₂-C₁₀ hydrocarbyl group;
X and X' are independently selected from C and N,
R⁵ is H if X is C, R^{5*'*} is H if X*'* is C, R⁵ is lone electron pair if X is N, R^{5*'*} is lone electron pair if X*'* is N, and
each of R³, R⁴, R^{3*'*}, R^{4*'*} is independently selected from H and C₆-C₁₀ aryl, with the proviso that
- neither both R³, R⁴ nor both R^{3*'*}, R^{4*'*} are aryl at the same time and
- if X is C, R³ and R⁴ are not H at the same time and if X*'* is C, R³ and R^{4*'*} are not H at the same time, or
both substituents on the same phenyl or pyridyl ring are hydrocarbyl groups linked with each other to form together a divalent C₄-C₁₀ hydrocarbyl group representing an anelated, substituted or unsubstituted, six-membered aromatic ring.

13. Compound according to claim 12, wherein R¹, R², R¹, R^{2*'*} is H, or R¹ with R² and R^{1*'*} with R^{2*'*} form anelated benzo-rings.

14. Compound according to claim 12 or 13, wherein R³ and R^{3*'*} are selected from H and phenyl, or R³ with R⁴ and R^{3*'*} with R^{4*'*} form anelated benzo-rings.

15. Compound according to any of claims 12-14, wherein R¹, R², R^{1*'*}, R^{2*'*} is H and X and X*'* is C.

## Patentansprüche

1. Verwendung einer Verbindung gemäß Formel 1: 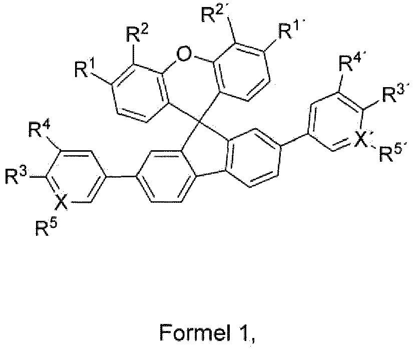
wobei jedes der R¹, R², R^{1'}, R^{2'} unabhängig ausgewählt ist aus H, C₁-C₆-Alkyl, C₁-C₆-Haloalkyl und C₆-C₁₀-Aryl oder beide Substituenten an dem gleichen aromatischen Ring des Xanthengerüsts Hydrocarbylgruppen sind, die derart miteinander verbunden sind, um zusammen eine anillierte divalente C₂-C₁₀-Hydrocarbylgruppe zu bilden;
X und X' unabhängig ausgewählt sind aus C und N,
R⁵ H ist, wenn X C ist, R^{5'} H ist, wenn X' C ist, R⁵ ein freies Elektronenpaar ist, wenn X N ist, R^{5'} ein freies Elektronenpaar ist, wenn X' N ist, und
jedes der R³, R⁴, R^{3'}, R^{4'} unabhängig ausgewählt ist aus H und C₆-C₁₀-Aryl, unter der Voraussetzung, dass
- weder beide der R³, R⁴ noch beide der R³, R^{4'} gleichzeitig Aryl sind und
- wenn X C ist, R³ und R⁴ nicht gleichzeitig H sind, und wenn X' C ist, R³ und R^{4'} nicht gleichzeitig H sind, oder
beide Substituenten an dem gleichen Phenyl- oder Pyridylring Hydrocarbylgruppen sind, die derart miteinander verbunden sind, um zusammen eine divalente C₄-C₁₀-Hydrocarbylgruppe zu bilden, die einen anillierten, substituierten oder unsubstituierten, sechsgliedrigen aromatischen Ring darstellt;
in einer Elektronentransportschicht oder in einer Elektroneninjektionsschicht, die in einer elektronischen Vorrichtung umfasst ist.

2. Verwendung nach Anspruch 1, wobei die elektronische Vorrichtung eine erste Elektrode und eine zweite Elektrode auf einem Substrat, eine lichtemittierende Schicht zwischen den ersten und zweiten Elektroden, eine erste Elektronentransportschicht zwischen der lichtemittierenden Schicht und der ersten Elektrode umfasst, wobei die erste Elektronentransportschicht die Verbindung gemäß der Formel 1 umfasst.

3. Verwendung nach Anspruch 2, wobei die erste Elektronentransportschicht aus der Verbindung gemäß der Formel 1 besteht.

4. Verwendung nach Anspruch 3, wobei die erste Elektronentransportschicht aus einer Verbindung einer einzigen Art besteht.

5. Verwendung nach einem der Ansprüche 2 bis 4, wobei die erste Elektronentransportschicht eine lochblockierende Schicht ist.

6. Verwendung nach einem der vorangehenden Ansprüche 2 bis 5, wobei die elektronische Vorrichtung ferner eine zweite Elektronentransportschicht zwischen der ersten Elektronentransportschicht und der ersten Elektrode umfasst.

7. Verwendung nach Anspruch 6, wobei die zweite Elektronentransportschicht eine Elektronentransportmatrix und einen elektrischen Dotanden umfasst.

8. Verwendung nach Anspruch 7, wobei die Elektronentransportmatrix in der zweiten Elektronentransportschicht eine Verbindung der Formel 1 umfasst.

9. Verwendung nach einem der Ansprüche 2 bis 8, wobei die erste Elektronentransportschicht einen elektrischen Dotanden umfasst.

10. Elektrisch dotiertes halbleitendes Material, das zumindest einen elektrischen Dotanden und eine Verbindung der Formel 1 umfasst 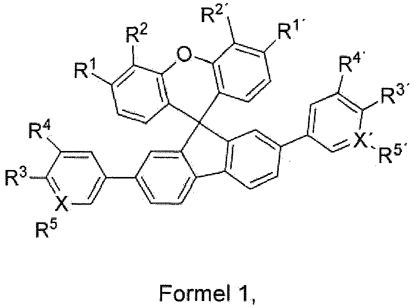
wobei jedes der R¹, R², R^{1'}, R^{2'} unabhängig ausgewählt ist aus H, C₁-C₆-Alkyl, C₁-C₆-Haloalkyl und C₆-C₁₀-Aryl oder beide Substituenten an dem gleichen aromatischen Ring des Xanthengerüsts Hydrocarbylgruppen sind, die derart miteinander verbunden sind, um zusammen eine anillierte divalente C₂-C₁₀-Hydrocarbylgruppe zu bilden;
X und X' unabhängig ausgewählt sind aus C und N,
R⁵ H ist, wenn X C ist, R^{5'} H ist, wenn X' C ist, R⁵ ein freies Elektronenpaar ist, wenn X N ist, R^{5'} ein freies Elektronenpaar ist, wenn X' N ist, und
jedes der R³, R⁴, R^{3'}, R^{4'} unabhängig ausgewählt ist aus H und C₆-C₁₀-Aryl, unter der Voraussetzung, dass
- weder beide der R³, R⁴ noch beide der R³, R^{4'} gleichzeitig Aryl sind und
- wenn X C ist, R³ und R⁴ nicht gleichzeitig H sind, und wenn X' C ist, R^{3'} und R^{4'} nicht gleichzeitig H sind, oder
beide Substituenten an dem gleichen Phenyl- oder Pyridylring Hydrocarbylgruppen sind, die derart miteinander verbunden sind, um zusammen eine divalente C₄-C₁₀-Hydrocarbylgruppe zu bilden, die einen anillierten, substituierten oder unsubstituierten, sechsgliedrigen aromatischen Ring darstellt.

11. Elektronische Vorrichtung, die ein elektrisch dotiertes Halbleitermaterial nach Anspruch 10 umfasst.

12. Verbindung, die eine Struktur gemäß der generischen Formel 1 hat 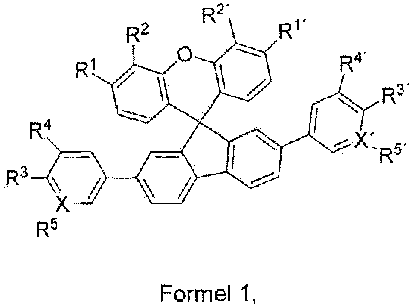
wobei jedes der R¹, R², R^{1'}, R^{2'} unabhängig ausgewählt ist aus H, C₁-C₆-Alkyl, C₁-C₆-Haloalkyl und C₆-C₁₀-Aryl oder beide Substituenten an dem gleichen aromatischen Ring des Xanthengerüsts Hydrocarbylgruppen sind, die derart miteinander verbunden sind, um zusammen eine anillierte divalente C₂-C₁₀-Hydrocarbylgruppe zu bilden;
X und X' unabhängig ausgewählt sind aus C und N,
R⁵ H ist, wenn X C ist, R^{5'} H ist, wenn X' C ist, R⁵ ein freies Elektronenpaar ist, wenn X N ist, R^{5'} ein freies Elektronenpaar ist, wenn X' N ist, und
jedes der R³, R⁴, R^{3'}, R^{4'} unabhängig ausgewählt ist aus H und C₆-C₁₀-Aryl, unter der Voraussetzung, dass
- weder beide der R³, R⁴ noch beide der R^{3'}, R^{4'} gleichzeitig Aryl sind und
- wenn X C ist, R³ und R⁴ nicht gleichzeitig H sind, und wenn X' C ist, R^{3'} und R^{4'} nicht gleichzeitig H sind, oder
beide Substituenten an dem gleichen Phenyl- oder Pyridylring Hydrocarbylgruppen sind, die derart miteinander verbunden sind, um zusammen eine divalente C₄-C₁₀ Hydrocarbylgruppe zu bilden, die einen anillierten, substituierten oder unsubstituierten, sechsgliedrigen aromatischen Ring darstellt.

13. Verbindung nach Anspruch 12, wobei R¹, R², R^{1'}, R^{2'} H sind, oder R¹ mit R² und R^{1'} mit R² anillierte Benzoringe bilden.

14. Verbindung nach Anspruch 12 oder 13, wobei R³ und R^{3'} ausgewählt sind aus H und Phenyl, oder R³ mit R⁴ und R^{3'} mit R^{4'} anillierte Benzoringe bilden.

15. Verbindung nach einem der Ansprüche 12 bis 14, wobei R¹, R², R^{1'}, R^{2'} H sind und X und X' C sind.

## Revendications

1. Utilisation d'un composé de formule 1 : 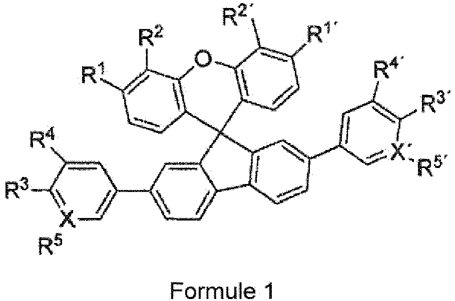
dans lequel chacun de R¹, R², R^{1'}, R^{2'} est indépendamment sélectionné parmi H, un groupe alkyle en C₁-C₆, un groupe haloalkyle en C₁-C₆, et un groupe aryle en C₆-C₁₀ ou les deux substituants sur le même cycle aromatique du squelette xanthène sont des groupes hydrocarbyle liés l'un à l'autre pour former ensemble un groupe hydrocarbyle en C₂-C₁₀ divalent annelé;
X et X' sont indépendamment sélectionnés parmi C et N,
R⁵ est H si X est C, R^{5'} est H si X' est C, R⁵ est un doublet d'électrons non liant si X est N, et R^{5'} est un doublet d'électrons non liant si X' est N, et
chacun de R³, R⁴, R^{3'}, R^{4'} est indépendamment sélectionné parmi H et un groupe aryle en C₆-C₁₀, à condition que
- ni R³ et R⁴, ni R^{3'} et R^{4'} ne soient un groupe aryle en même temps, et
- si X est C, R³ et R⁴ ne soient pas H en même temps, et si X' est C, R^{3'} et R^{4'} ne soient pas H en même temps, ou
les deux substituants sur le même cycle phényle ou pyridyle sont des groupes hydrocarbyle liés l'un à l'autre pour former ensemble un groupe hydrocarbyle en C₄-C₁₀ divalent représentant un cycle aromatique à six chaînons, annelé, substitué ou non substitué ;
dans une couche de transport d'électrons ou dans une couche d'injection d'électrons comprise dans un dispositif électronique.

2. Utilisation selon la revendication 1, dans laquelle le dispositif électronique comprend une première électrode et une seconde électrode sur un substrat, une couche d'émission de lumière entre les première et seconde électrodes, une première couche de transport d'électrons entre la couche d'émission de lumière et la première électrode, ladite première couche de transport d'électrons comprenant le composé de formule 1.

3. Utilisation selon la revendication 2, dans laquelle la première couche de transport d'électrons est constituée du composé de formule 1.

4. Utilisation selon la revendication 3, dans laquelle la première couche de transport d'électrons est constituée d'un composé mono-espèce.

5. Utilisation selon l'une quelconque des revendications 2 à 4, dans laquelle la première couche de transport d'électrons est une couche de blocage de trous.

6. Utilisation selon l'une quelconque des revendications 2 à 5 précédentes, dans laquelle le dispositif électronique comprend en outre une seconde couche de transport d'électrons entre la première couche de transports d'électrons et la première électrode.

7. Utilisation selon la revendication 6, dans laquelle la seconde couche de transport d'électrons comprend une matrice de transport d'électrons et un dopant électrique.

8. Utilisation selon la revendication 7, dans laquelle la matrice de transport d'électrons dans la seconde couche de transport d'électrons comprend le composé de formule 1.

9. Utilisation selon l'une quelconque des revendications 2 à 8, dans laquelle la première couche de transport d'électrons comprend un dopant électrique.

10. Matériau semi-conducteur électriquement dopé comprenant au moins un dopant électrique et le composé de formule 1 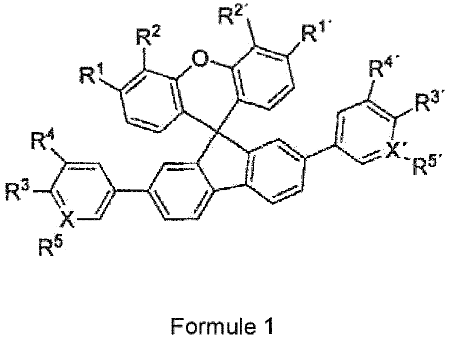
dans lequel chacun de R¹, R², R^{1'}, R^{2'} est indépendamment sélectionné parmi H, un groupe alkyle en C₁-C₆, un groupe haloalkyle en C₁-C₆, et un groupe aryle en C₆-C₁₀ ou les deux substituants sur le même cycle aromatique du squelette xanthène sont des groupes hydrocarbyle liés l'un à l'autre pour former ensemble un groupe hydrocarbyle en C₂-C₁₀ divalent annelé ;
X et X' sont indépendamment sélectionnés parmi C et N,
R⁵ est H si X est C, R^{5'} est H si X' est C, R⁵ est un doublet d'électrons non liant si X est N, et R^{5'} est un doublet d'électrons non liant si X' est N, et
chacun de R³, R⁴, R^{3'}, R^{4'} est indépendamment sélectionné parmi H et un groupe aryle en C₆-C₁₀ à condition que
- ni R³ et R⁴, ni R^{3'} et R^{4'} ne soient un groupe aryle en même temps, et
- si X est C, R³ et R⁴ ne soient pas H en même temps, et si X' est C, R^{3'} et R^{4'} ne soient pas H en même temps, ou
les deux substituants sur le même cycle phényle ou pyridyle sont des groupes hydrocarbyle liés l'un à l'autre pour former ensemble un groupe hydrocarbyle en C₄-C₁₀ divalent représentant un cycle aromatique à six chaînons, annelé, substitué ou non substitué.

11. Dispositif électronique comprenant le matériau semi-conducteur électriquement dopé selon la revendication 10.

12. Composé répondant à la structure de formule générale 1 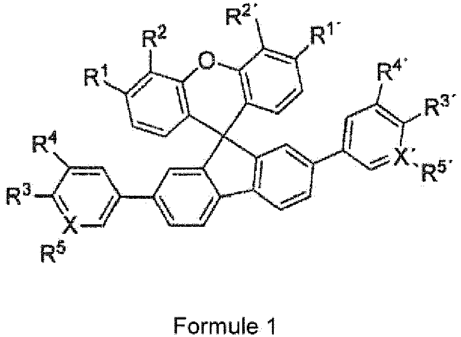
dans lequel chacun de R¹, R², R¹, R² est indépendamment sélectionné parmi H, une groupe alkyle en C₁-C₆, un groupe haloalkyle en C₁-C₆, et un groupe aryle en C₆-C₁₀ ou les deux substituants sur le même cycle aromatique du squelette xanthène sont des groupes hydrocarbyle liés l'un à l'autre pour former ensemble un groupe hydrocarbyle en C₂-C₁₀ divalent annelé;
X et X' sont indépendamment sélectionnés parmi C et N,
R⁵ est H si X est C, R^{5'} est H si X' est C, R⁵ est un doublet d'électrons non liant si X est N, et R⁵ est un doublet d'électrons non liant si X' est N, et
chacun de R³, R⁴, R^{3'}, R^{4'} est indépendamment sélectionné parmi H et un groupe aryle en C₆-C₁₀, à condition que
- ni R³ et R⁴, ni R^{3'} et R^{4'} ne soient un groupe aryle en même temps, et
- si X est C, R³ et R⁴ ne soient pas H en même temps, et si X' est C, R^{3'} et R^{4'} ne soient pas H en même temps, ou
les deux substituants sur le même cycle phényle ou pyridyle sont des groupes hydrocarbyle liés l'un à l'autre pour former ensemble un groupe hydrocarbyle en C₄-C₁₀ divalent représentant un cycle aromatique à six chaînons, annelé, substitué ou non substitué.

13. Composé selon la revendication 12, dans lequel R¹, R², R^{1'} et R^{2'} sont H, ou R¹ avec R² et R¹ avec R^{2'} forment des cycles benzo annelés.

14. Composé selon la revendication 12 ou 13, dans lequel R³ et R^{3'} sont sélectionnés parmi H et un groupe phényle, ou R³ avec R⁴ et R^{3'} avec R^{4'} forment des cycles benzo annelés.

15. Composé selon les revendications 12 à 14, dans lequel R¹, R², R^{1'}, R^{2'} sont H, et X et X' sont C.
